# EUROPEAN PATENT APPLICATION

(11) **EP 4 115 889 A1**
(43) Date of publication of application: **11.01.2023**
(21) Application number: 21757758.4
(22) Date of filing: 19.02.2021
(51) Int. Cl.: A61K 31/7105, A61K 9/06, A61K 47/42, A61P 1/02, A61P 19/08, A61P 43/00, C12N 15/113

(54) **MEDICINE CONTAINING USAG-1-TARGETING RNA MOLECULE FOR TOOTH REGENERATION THERAPY**

(30) Priority: 21.02.2020 JP 2020028547
(71) Applicant: Kyoto University, Kyoto-shi, Kyoto 606-8501 (JP); University of Fukui, Fukui-shi, Fukui 910-8507 (JP); Aichi Prefecture, Aichi 460-8501 (JP)
(72) Inventor: TAKAHASHI, Katsu, Kyoto-shi, Kyoto 606-8501 (JP); BESSHO, Kazuhisa, Kyoto-shi, Kyoto 606-8501 (JP); TABATA, Yasuhiko, Kyoto-shi, Kyoto 606-8501 (JP); SUGAI, Manabu, Yoshida-gun, Fukui 910-1193 (JP); TOKITA, Yoshihito, Kasugai-shi, Aichi 480-0392 (JP)
(74) Representative: Hoffmann Eitle
(86) International application number: PCT/JP2021/006296
(87) International publication number: WO 2021/167056

(57) **Abstract**

Provided is a medicinal composition to be topically administered for tooth regeneration therapy, said composition comprising an RNA molecule targeting USAG-1 or a nucleic acid molecule capable of yielding the RNA molecule and a pharmaceutically acceptable carrier.

## Description

### Technical Field

The present invention relates to a therapeutic agent containing an RNA molecule targeting USAG-1 for tooth regeneration, in particular, tooth agenesis.

### Background Art

In the majority of patients, acquired diseases such as dental caries and periodontal disease result in tooth agenesis (patients with loss of teeth). As high as 1% incidence rate of congenital tooth agenesis is also reported. Currently, the only treatment method for missing teeth is prosthetic treatment which includes dental implants and dentures, and there is no fundamental treatment method. Numerous studies of tooth regeneration using tissue engineering approaches have been reported. Various cells such as stem cells (Non-Patent Literature 1) are used as cell sources. In addition, in order to allow teeth made in vitro to function in the oral cavity, an "organ primordium method" (Non-Patent Literature 2), a cell manipulation technology for regenerating a dental organ primordium (the rudiment of the organ) in a collagen gel, has been reported. However, owing to cost and safety problems for securing cell sources, tissue engineering approaches have not reached clinical application.

A large number of causative genes for congenital tooth agenesis have been identified, and many of them are common to both human and mouse. For example, RUNX2, MSX1, EDA, WNT10A, PAX9, AXIN2 etc. are known as the causative genes for congenital tooth agenesis. Congenital tooth agenesis is caused by tooth development stopped prematurely due to defect in the causative gene and suppression of the function of the causative gene.

### Citation List

### Non-Patent Literature

Non-Patent Literature 1: Ohazama A. et al., J Denr Res, 2004 Jul; 83(7): 518-22
Non-Patent Literature 2: Nakao K. et al., Nat Methods, 2007 Mar; 4(3): 227-30

### Summary of Invention

### Problem to be solved by Invention

From a therapeutic viewpoint, the present inventors conceived of a novel treatment method to treat congenital tooth agenesis. The new approach promotes differentiation induction from the state of tooth development that has been stopped prematurely to form a complete tooth. Object of the present invention is to provide a technique for tooth regeneration therapy which comprises utilizing the differentiation induction inherent in a tooth organ, instead of utilizing surgical tissue transplantation.

### Solution for Problem

As a result of diligent research, the present inventors found that when an USAG-1 gene-targeting siRNA and a Runx2-targeting siRNA were subjected to a subrenal capsule assay of a mouse mandible, a reduced number of teeth caused by the knockdown of Runx2 was recovered by the knockdown of USAG-1. Furthermore, when an USAG-1 gene-targeting siRNA was sujected to a subrenal capsule assay of a Runx2-knockout mouse mandible, a tooth-like structure was observed. Thus it was found that tooth agenesis can be treated using an USAG-1 gene-targeting RNA molecule. Therefore, the present invention was completed.

Specifically, the present invention provides:
[1] a pharmaceutical composition for topical administration for tooth regeneration therapy, comprising an RNA molecule targeting USAG-1 or a nucleic acid molecule that produces the RNA molecule, and a pharmaceutically acceptable carrier,
[2] the pharmaceutical composition according to [1], wherein the RNA molecule is selected from the group consisting of a siRNA, a shRNA, an antisense RNA, a miRNA, and a ribozyme,
[3] the pharmaceutical composition according to [2], wherein the RNA molecule is a siRNA or a shRNA,
[4] the pharmaceutical composition according to [3], wherein the siRNA or shRNA comprises:
   (1) a sense strand comprising a nucleotide sequence shown in SEQ ID NO: 1, or a nucleotide sequence that differs from the nucleotide sequence shown in SEQ ID NO: 1 by deletion, substitution, insertion and/or addition of one to several nucleotides, and an antisense strand comprising a nucleotide sequence shown in SEQ ID NO: 2, or a nucleotide sequence that differs from the nucleotide sequence shown in SEQ ID NO: 2 by deletion, substitution, insertion and/or addition of one to several nucleotides, or
   (2) a sense strand comprising a nucleotide sequence shown in SEQ ID NO: 3, or a nucleotide sequence that differs from the nucleotide sequence shown in SEQ ID NO: 3 by deletion, substitution, insertion and/or addition of one to several nucleotides, and an antisense strand comprising a nucleotide sequence shown in SEQ ID NO: 4, or a nucleotide sequence that differs from the nucleotide sequence shown in SEQ ID NO: 4 by deletion, substitution, insertion and/or addition of one to several nucleotides,
[5] the pharmaceutical composition according to [2], wherein the RNA molecule is an antisense RNA,
[6] the pharmaceutical composition according to [5], wherein the antisense RNA comprises:
   (1) a nucleotide sequence shown in SEQ ID NO: 2, or a nucleotide sequence that differs from the nucleotide sequence shown in SEQ ID NO: 2 by deletion, substitution, insertion and/or addition of one to several nucleotides, or
   (2) a nucleotide sequence shown in SEQ ID NO: 4, or a nucleotide sequence that differs from the nucleotide sequence shown in SEQ ID NO: 4 by deletion, substitution, insertion and/or addition of one to several nucleotides,
[7] the pharmaceutical composition according to any one of [1] to [6], wherein the carrier is selected from the group consisting of collagen, gelatin, gelatin hydrogel, polylactic acid, a poly(lactic acid-co-glycolic acid) copolymer (PLGA), an in situ gelling system comprising polymethacrylic acid (PMA) and polyethylene glycol (PEG) and hydroxypropylmethylcellulose, a poly(lactic acid)-poly(ethylene glycol) copolymer (PLA-PEG), poly(2-aminoethyl propylene phosphate), poly(α-(4-aminobutyl)-L-glycolic acid) (PAGA), a poloxamer, an ethylene-co-vinyl acetate copolymer (EVAc), a silk elastin-like polymer (SELP), denatured collagen-PLGA, atelocollagen, a liposome, a nanoparticle comprising cationic dextrin, a nanoparticle comprising PEG, a nanoparticle comprising transferrin, and a nanoparticle comprising adamantan,
[8] the pharmaceutical composition according to [7], wherein the carrier is cationized gelatin, cationized gelatin hydrogel, or a cationized gelatin microsphere,
[9] the pharmaceutical composition according to any one of [1] to [8], wherein the tooth regeneration therapy is a treatment of congenital or acquired tooth agenesis, or regeneration of a missing tooth,
[10] a use of an RNA molecule targeting USAG-1 or a nucleic acid molecule that produces the RNA molecule, and a pharmaceutically acceptable carrier in the manufacture of a pharmaceutical composition for topical administration for tooth regeneration therapy,
[11] the use according to [10], wherein the RNA molecule is selected from the group consisting of a siRNA, a shRNA, an antisense RNA, a miRNA, and a ribozyme,
[12] the use according to [11], wherein the RNA molecule is a siRNA or a shRNA,
[13] the use according to [12], wherein the siRNA or shRNA comprises:
   (1) a sense strand comprising a nucleotide sequence shown in SEQ ID NO: 1, or a nucleotide sequence that differs from the nucleotide sequence shown in SEQ ID NO: 1 by deletion, substitution, insertion and/or addition of one to several nucleotides, and an antisense strand comprising a nucleotide sequence shown in SEQ ID NO: 2, or a nucleotide sequence that differs from the nucleotide sequence shown in SEQ ID NO: 2 by deletion, substitution, insertion and/or addition of one to several nucleotides, or
   (2) a sense strand comprising a nucleotide sequence shown in SEQ ID NO: 3, or a nucleotide sequence that differs from the nucleotide sequence shown in SEQ ID NO: 3 by deletion, substitution, insertion and/or addition of one to several nucleotides, and an antisense strand comprising a nucleotide sequence shown in SEQ ID NO: 4, or a nucleotide sequence that differs from the nucleotide sequence shown in SEQ ID NO: 4 by deletion, substitution, insertion and/or addition of one to several nucleotides,
[14] the use according to [11], wherein the RNA molecule is an antisense RNA,
[15] the use according to [11], wherein the antisense RNA comprises:
   (1) a nucleotide sequence shown in SEQ ID NO: 2, or a nucleotide sequence that differs from the nucleotide sequence shown in SEQ ID NO: 2 by deletion, substitution, insertion and/or addition of one to several nucleotides, or
   (2) a nucleotide sequence shown in SEQ ID NO: 4, or a nucleotide sequence that differs from the nucleotide sequence shown in SEQ ID NO: 4 by deletion, substitution, insertion and/or addition of one to several nucleotides,
[16] the use according to any one of [10] to [15], wherein the carrier is selected from the group consisting of collagen, gelatin, gelatin hydrogel, polylactic acid, a poly(lactic acid-co-glycolic acid) copolymer (PLGA), an in situ gelling system comprising polymethacrylic acid (PMA) and polyethylene glycol (PEG) and hydroxypropylmethylcellulose, a poly(lactic acid)-poly(ethylene glycol) copolymer (PLA-PEG), poly(2-aminoethyl propylene phosphate), poly(α-(4-aminobutyl)-L-glycolic acid) (PAGA), a poloxamer, an ethylene-co-vinyl acetate copolymer (EVAc), a silk elastin-like polymer (SELP), denatured collagen-PLGA, atelocollagen, a liposome, a nanoparticle comprising cationic dextrin, a nanoparticle comprising PEG, a nanoparticle comprising transferrin, and a nanoparticle comprising adamantan,
[17] the use according to [16], wherein the carrier is cationized gelatin, cationized gelatin hydrogel, or a cationized gelatin microsphere,
[18] the use according to any one of [10] to [17], wherein the tooth regeneration therapy is a treatment of congenital or acquired tooth agenesis, or regeneration of a missing tooth,
[19] a use of an RNA molecule targeting USAG-1 or a nucleic acid molecule that produces the RNA molecule for tooth regeneration therapy,
[20] the use according to [19], in combination with a pharmaceutically acceptable carrier,
[21] the use according to [19] or [20], which is topical use,
[22] the use according to any one of [19] to [21], wherein the RNA molecule is selected from the group consisting of a siRNA, a shRNA, an antisense RNA, a miRNA, and a ribozyme,
[23] the use according to [22], wherein the RNA molecule is a siRNA or a shRNA,
[24] the use according to [23], wherein the siRNA or shRNA comprises:
   (1) a sense strand comprising a nucleotide sequence shown in SEQ ID NO: 1, or a nucleotide sequence that differs from the nucleotide sequence shown in SEQ ID NO: 1 by deletion, substitution, insertion and/or addition of one to several nucleotides, and an antisense strand comprising a nucleotide sequence shown in SEQ ID NO: 2, or a nucleotide sequence that differs from the nucleotide sequence shown in SEQ ID NO: 2 by deletion, substitution, insertion and/or addition of one to several nucleotides, or
   (2) a sense strand comprising a nucleotide sequence shown in SEQ ID NO: 3, or a nucleotide sequence that differs from the nucleotide sequence shown in SEQ ID NO: 3 by deletion, substitution, insertion and/or addition of one to several nucleotides, and an antisense strand comprising a nucleotide sequence shown in SEQ ID NO: 4, or a nucleotide sequence that differs from the nucleotide sequence shown in SEQ ID NO: 4 by deletion, substitution, insertion and/or addition of one to several nucleotides,
[25] the use according to [22], wherein the RNA molecule is an antisense RNA,
[26] the use according to [25], wherein the antisense RNA comprises:
   (1) a nucleotide sequence shown in SEQ ID NO: 2, or a nucleotide sequence that differs from the nucleotide sequence shown in SEQ ID NO: 2 by deletion, substitution, insertion and/or addition of one to several nucleotides, or
   (2) a nucleotide sequence shown in SEQ ID NO: 4, or a nucleotide sequence that differs from the nucleotide sequence shown in SEQ ID NO: 4 by deletion, substitution, insertion and/or addition of one to several nucleotides,
[27] the use according to any one of [20] to [26], wherein the carrier is selected from the group consisting of collagen, gelatin, gelatin hydrogel, polylactic acid, a poly(lactic acid-co-glycolic acid) copolymer (PLGA), an in situ gelling system comprising polymethacrylic acid (PMA) and polyethylene glycol (PEG) and hydroxypropylmethylcellulose, a poly(lactic acid)-poly(ethylene glycol) copolymer (PLA-PEG), poly(2-aminoethyl propylene phosphate), poly(α-(4-aminobutyl)-L-glycolic acid) (PAGA), a poloxamer, an ethylene-co-vinyl acetate copolymer (EVAc), a silk elastin-like polymer (SELP), denatured collagen-PLGA, atelocollagen, a liposome, a nanoparticle comprising cationic dextrin, a nanoparticle comprising PEG, a nanoparticle comprising transferrin, and a nanoparticle comprising adamantan,
[28] the use according to [27], wherein the carrier is cationized gelatin, cationized gelatin hydrogel, or a cationized gelatin microsphere,
[29] the use according to any one of [19] to [28], wherein the tooth regeneration therapy is a treatment of congenital or acquired tooth agenesis, or regeneration of a missing tooth,
[30] a method for tooth regeneration therapy, the method comprising topically administering an RNA molecule targeting USAG-1 or a nucleic acid molecule that produces the RNA molecule in combination with a pharmaceutically acceptable carrier, to a tooth-missing site or a tooth-formation site,
[31] the method according to [30], wherein the RNA molecule is selected from the group consisting of siRNA, shRNA, antisense RNA, miRNA, and ribozyme,
[32] the method according to [31], wherein the RNA molecule is a siRNA or a shRNA,
[33] the method according to [32], wherein the siRNA or shRNA comprises:
   (1) a sense strand comprising a nucleotide sequence shown in SEQ ID NO: 1, or a nucleotide sequence that differs from the nucleotide sequence shown in SEQ ID NO: 1 by deletion, substitution, insertion and/or addition of one to several nucleotides, and an antisense strand comprising a nucleotide sequence shown in SEQ ID NO: 2, or a nucleotide sequence that differs from the nucleotide sequence shown in SEQ ID NO: 2 by deletion, substitution, insertion and/or addition of one to several nucleotides, or
   (2) a sense strand comprising a nucleotide sequence shown in SEQ ID NO: 3, or a nucleotide sequence that differs from the nucleotide sequence shown in SEQ ID NO: 3 by deletion, substitution, insertion and/or addition of one to several nucleotides, and an antisense strand comprising a nucleotide sequence shown in SEQ ID NO: 4, or a nucleotide sequence that differs from the nucleotide sequence shown in SEQ ID NO: 4 by deletion, substitution, insertion and/or addition of one to several nucleotides,
[34] the method according to [31], wherein the RNA molecule is an antisense RNA,
[35] the method according to [34], wherein the antisense RNA comprises:
   (1) a nucleotide sequence shown in SEQ ID NO: 2, or a nucleotide sequence that differs from the nucleotide sequence shown in SEQ ID NO: 2 by deletion, substitution, insertion and/or addition of one to several nucleotides, or
   (2) a nucleotide sequence shown in SEQ ID NO: 4, or a nucleotide sequence that differs from the nucleotide sequence shown in SEQ ID NO: 4 by deletion, substitution, insertion and/or addition of one to several nucleotides,
[36] the method according to any one of [30] to [35], wherein the carrier is selected from the group consisting of collagen, gelatin, gelatin hydrogel, polylactic acid, a poly(lactic acid-co-glycolic acid) copolymer (PLGA), an in situ gelling system comprising polymethacrylic acid (PMA) and polyethylene glycol (PEG) and hydroxypropylmethylcellulose, a poly(lactic acid)-poly(ethylene glycol) copolymer (PLA-PEG), poly(2-aminoethyl propylene phosphate), poly(α-(4-aminobutyl)-L-glycolic acid) (PAGA), a poloxamer, an ethylene-co-vinyl acetate copolymer (EVAc), a silk elastin-like polymer (SELP), denatured collagen-PLGA, atelocollagen, a liposome, a nanoparticle comprising cationic dextrin, a nanoparticle comprising PEG, a nanoparticle comprising transferrin, and a nanoparticle comprising adamantan,
[37] the method according to [36], wherein the carrier is cationized gelatin, cationized gelatin hydrogel, or a cationized gelatin microsphere, and
[38] the method according to any one of [30] to [37], wherein the tooth regeneration therapy is a treatment of congenital or acquired tooth agenesis, or regeneration of a missing tooth.

### Effects of the Invention

Tooth regeneration including treatment of congenital and acquired tooth agenesis becomes possible by topical administration of the pharmaceutical composition of the present invention. The treatment with the pharmaceutical composition of the present invention can be clinically applied as a tooth regenerative therapy in a general dental and oral surgical approach such as conventional tooth extraction, orthodontics, and tooth transplantation.

### Brief Description of Drawings

[FIG. 1A] Figure 1A shows the USAG-1 gene knockdown effects of siRNAs targeting mouse USAG-1 in mouse enamel epithelial stem cell-like strain mHAT9D. The upper figure shows results of electrophoresis. The lower figure shows the intensities of electrophoresis bands of serial dilutions (1/10, 1/30 and 1/90) in each siRNA (#304 or #903) administration group, wherein the intensities of control bands are defined as 1.
[FIG. 1B] Figure 1B shows the USAG-1 gene knockdown effects of siRNAs targeting mouse USAG-1 in a mouse mandibular organ culture. The upper figure shows results of electrophoresis. The lower figure shows the intensities of electrophoresis bands of serial dilutions (1/10, 1/30 and 1/90) in each siRNA (#304 or #903) administration group, wherein the intensities of control bands are defined as 1.
[FIG. 1C] Figure 1C shows the effects of siRNAs on histological evaluations at the most advanced development stage of tooth germs and the number of tooth germs in a wild-type mouse mandibular organ culture.
[FIG. 2A] Figure 2A is a scheme of subrenal capsule assay. In the figure, "M" indicates a lateral mandible of an E10 mouse, "G" indicates cationized gelatin impregnated with a siRNA, and "A" indicates agarose for maintaining a transplantation space.
[FIG. 2B] Figure 2B shows microscopic photographs of hematoxylin and eosin (HE)-stained sections of wild-type mouse lateral mandibles that were subjected to a subrenal capsule assay of nude mice (KSN/Slc). The subrenal capsule assay was performed in two groups: a group of transplantation together with a cationized gelatin sheet impregnated with PBS and a group of transplantation together with no cationized gelatin sheet (only lateral mandible).
[FIG. 2C] Figure 2C shows histological sections evaluated by HE staining (upper figure) and immunostaining (lower figure) 10 days after the subrenal capsule assay. The magnification in the left figures is 200 times, and the magnification in the right figures is 1000 times. Positive cells were immunostained with Alexa Fluor 488.
[FIG. 3] Figure 3 shows the effects of siRNAs in a subrenal capsule assay of a wild-type mouse mandible.
[FIG. 4A] Figure 4A shows the effects of cationized gelatin impregnated with USAG-1 siRNA in a subrenal capsule assay of a Runx2 KO mouse mandible.
[FIG. 4B] Figure 4B shows the effects of cationized gelatin impregnated with USAG-1 siRNA in a subrenal capsule assay of a Runx2 KO mouse mandible.
[FIG. 5A] Figure 5A shows the effects of cationized gelatin impregnated with USAG-1 siRNA in a subrenal capsule assay of a Runx2 KO mouse mandible.
[FIG. 5B] Figure 5B shows the effects of cationized gelatin impregnated with USAG-1 siRNA in a subrenal capsule assay of a Runx2 KO mouse mandible.
[FIG. 6A] Figure 6A shows the USAG-1 gene knockdown effects of a siRNA targeting human USAG-1. The upper figure shows results of electrophoresis. The lower figure shows the intensities of electrophoresis bands of serial dilutions (1/10, 1/30 and 1/90) in a siRNA administration group, wherein the intensities of control bands are defined as 1.
[FIG. 6B] Figure 6B shows the USAG-1 gene knockdown effects of a siRNA targeting human USAG-1. The upper figure shows results of electrophoresis. The lower figure shows the intensities of electrophoresis bands of serial dilutions (1/10, 1/30 and 1/90) in a siRNA administration group, wherein the intensities of control bands are defined as 1.

### Mode for carrying out the Invention

USAG-1 (Uterine Sensitization Associated Gene-1) is a bone morphogenetic protein (BMP) antagonist and a Wnt antagonist, and is also called Sostdc-1, Ectodin, or Wise. It is known that in USAG-1 deficient model mice, an increase in BMP signaling is observed, leading to the formation of supernumerary teeth. The present inventors crossed a Runx2-, Msx1-, Eda-, or Wnt10a-deficient mouse, which is a model mouse for congenital tooth agenesis, with a USAG-1 gene-deficient mouse, which is a model mouse for supernumerary teeth (teeth exceeding the normal number of teeth), to produce a double-knockout mouse. As a result of analysis of the double-knockout mouse, it was found that tooth formation was recovered in all the model mice for congenital tooth agenesis. Thus it was suggested that inhibition of USAG-1 could treat tooth agenesis.

Therefore, the present inventors studied the effectiveness of RNA molecule administration for the purpose of inhibiting USAG-1. As a result, it was shown that the expression of USAG-1 can be inhibited by administration of a RNA molecule targeting USAG-1, and thereby the number of teeth can be recovered.

Thus, one aspect of the present invention provides a pharmaceutical composition for topical administration for tooth regeneration therapy, comprising an RNA molecule targeting USAG-1 and a pharmaceutically acceptable carrier (hereinafter also referred to as "the pharmaceutical composition of the present disclosure").

As used herein, the "RNA molecule" is a double-stranded or single-stranded RNA molecule capable of acting on the mRNA of a gene of interest. Examples thereof include a siRNA (small interfering RNA), a shRNA (short hairpin RNA), an antisense RNA, a miRNA (microRNA), and a ribozyme. The RNA molecule may be chemically modified, and may be preferably modified for improving intracellular uptake efficiency, improving the ability to bind to mRNA, improving knockdown efficiency of a target gene, suppressing off-target effects, improving specificity for a target gene, improving the stability of the RNA molecule (for example, conferring nuclease resistance), and/or reducing the cytotoxicity of the RNA molecule. Examples of the chemical modification include, but not limited to, phosphorothioation (S-modification) of a phosphate moiety, modification at the 2' position of a sugar moiety (for example, 2'-fluoro, 2'-O-methyl, 2'-O-methoxyethyl sugar), cross-linking modification of a sugar moiety, for example, chemical cross-linking modification at the 2'-position and 4'-position of a sugar moiety [for example, 2',4'-BNA (2',4'-Bridged Nucleic Acid), BNA^{COC}, BNA^{NC} , ENA (2'-O, 4' -C-Ethylene-bridged Nucleic Acid), cEt BNA, etc.], and modification of a sugar moiety with a morpholine ring (morpholino nucleic acid). Further, the RNA molecule may partially contain a DNA sequence. For example, the RNA molecule comprises a nucleotide sequence complementary to a mRNA sequence of a gene of interest or a partial sequence thereof, and binds (preferably specifically binds) to the mRNA sequence of the gene of interest or a partial sequence thereof. Therefore, the RNA molecule contained in the pharmaceutical composition of the present disclosure preferably comprises a nucleotide sequence complementary to a mRNA sequence of a USAG-1 gene or a partial sequence of the mRNA sequence. Various RNA molecules and their modifications have been well studied in the art, and a person skilled in the art can appropriately design and prepare RNA molecules based on a gene of interest.

As used herein with respect to the RNA molecule, the term "target" or a derivative thereof means that the RNA molecule acts on a messenger RNA (mRNA) of a gene of interest, for example, the RNA molecule binds (preferably, specifically binds) to the mRNA sequence or a partial sequence thereof of the gene of interest to exert an action on the mRNA. As used herein, the "action" is any action that brings about inhibition of expression of a protein encoded by a gene of interest. Examples of the action include, but not limited to, induction of mRNA degradation, and inhibition of mRNA translation. The mRNA comprises a 5' untranslated region, a coding region, and a 3' untranslated region.

As used herein with respect to expression, the term "inhibition" means that the expression level or function of a protein of interest is reduced as compared to a control, or the protein is not expressed or does not function. As used herein, the term "control" means a subject to which the RNA molecule described herein has not been administered.

Therefore, as used herein, the "RNA molecule targeting USAG-1" is an RNA molecule that acts on the mRNA of a USAG-1 gene and thereby inhibits the expression of UASG-1. The RNA molecule inhibits the production of USAG-1, resulting in tooth regeneration. Since the pharmaceutical composition of the present disclosure inhibits the production of USAG-1 protein itself, the expected therapeutic effect is exerted by a mechanism different from a technique using an antibody or the like that blocks the action of the protein after the production of the protein.

The "siRNA" is a double-stranded RNA comprising the same sequence as the mRNA sequence of a target gene, and a complementary sequence thereto. The siRNA may have a single-stranded overhang at one or both ends. The overhang may be a single-stranded region consisting of, for example, 1 to 10 bases, preferably 1 to 4 bases. The siRNA may comprise a double-stranded region consisting of, for example, 15 to 40 base pairs, preferably 18 to 35 base pairs, and more preferably 20 to 25 base pairs.

When the RNA molecule contained in the pharmaceutical composition of the present disclosure is a siRNA, the siRNA preferably comprises the same nucleotide sequence as the mRNA sequence of a USAG-1 gene or a partial sequence thereof, and a complementary nucleotide sequence thereto. As an example, the siRNA comprises a sense strand comprising a nucleotide sequence shown in SEQ ID NO: 1, or a nucleotide sequence that differs from the nucleotide sequence shown in SEQ ID NO: 1 by deletion, substitution, insertion and/or addition of one to several nucleotides, and an antisense strand comprising a nucleotide sequence shown in SEQ ID NO: 2, or a nucleotide sequence that differs from the nucleotide sequence shown in SEQ ID NO: 2 by deletion, substitution, insertion and/or addition of one to several nucleotides. As another example, the siRNA comprises a sense strand comprising a nucleotide sequence shown in SEQ ID NO: 3, or a nucleotide sequence that differs from the nucleotide sequence shown in SEQ ID NO: 3 by deletion, substitution, insertion and/or addition of one to several nucleotides, and an antisense strand comprising a nucleotide sequence shown in SEQ ID NO: 4, or a nucleotide sequence that differs from the nucleotide sequence shown in SEQ ID NO: 4 by deletion, substitution, insertion and/or addition of one to several nucleotides.

As a preferred example, the siRNA comprises a sense strand comprising a nucleotide sequence shown in SEQ ID NO: 1, or a nucleotide sequence that differs from the nucleotide sequence shown in SEQ ID NO: 1 by deletion, substitution, insertion and/or addition of one to three nucleotides, and an antisense strand comprising a nucleotide sequence shown in SEQ ID NO: 2, or a nucleotide sequence that differs from the nucleotide sequence shown in SEQ ID NO: 2 by deletion, substitution, insertion and/or addition of one to three nucleotides. As another preferred example, the siRNA comprises a sense strand comprising a nucleotide sequence shown in SEQ ID NO: 3, or a nucleotide sequence that differs from the nucleotide sequence shown in SEQ ID NO: 3 by deletion, substitution, insertion and/or addition of one to three nucleotides, and an antisense strand comprising a nucleotide sequence shown in SEQ ID NO: 4, or a nucleotide sequence that differs from the nucleotide sequence shown in SEQ ID NO: 4 by deletion, substitution, insertion and/or addition of one to three nucleotides.

As a more preferred example, the siRNA comprises a sense strand comprising a nucleotide sequence shown in SEQ ID NO: 1, or a nucleotide sequence that differs from the nucleotide sequence shown in SEQ ID NO: 1 by deletion, substitution, insertion and/or addition of one or two nucleotides, and an antisense strand comprising a nucleotide sequence shown in SEQ ID NO: 2, or a nucleotide sequence that differs from the nucleotide sequence shown in SEQ ID NO: 2 by deletion, substitution, insertion and/or addition of one or two nucleotides. As another more preferred example, the siRNA comprises a sense strand comprising a nucleotide sequence shown in SEQ ID NO: 3, or a nucleotide sequence that differs from the nucleotide sequence shown in SEQ ID NO: 3 by deletion, substitution, insertion and/or addition of one or two nucleotides, and an antisense strand comprising a nucleotide sequence shown in SEQ ID NO: 4, or a nucleotide sequence that differs from the nucleotide sequence shown in SEQ ID NO: 4 by deletion, substitution, insertion and/or addition of one or two nucleotides.

As a still more preferred example, the siRNA comprises a sense strand comprising a nucleotide sequence shown in SEQ ID NO: 1, and an antisense strand comprising a nucleotide sequence shown in SEQ ID NO: 2. As another still more preferred example, the siRNA comprises a sense strand comprising a nucleotide sequence shown in SEQ ID NO: 3, and an antisense strand comprising a nucleotide sequence shown in SEQ ID NO: 4.

The "shRNA" is a single-stranded RNA having a hairpin shape comprising a stem consisting of a double-stranded region and a loop consisting of a single-stranded region. The shRNA may be, for example, about 30 to 110 bases in length, preferably 40 to 100 bases in length, more preferably 50 to 90 bases in length. The siRNA may be a shRNA capable of forming a double strand in cells after administration. For example, the siRNA may be a shRNA comprising a stem of a siRNA moiety and a loop of an arbitrary sequence. The arbitrary loop sequence may be, for example, 1 to 30 bases in length, preferably 1 to 25 bases in length, more preferably 5 to 20 bases in length

When the RNA molecule contained in the pharmaceutical composition of the present disclosure is a shRNA, the shRNA preferably comprises the same nucleotide sequence as the mRNA sequence of a USAG-1 gene or a partial sequence thereof, and a complementary nucleotide sequence thereto. As an example, the stem part of the shRNA comprises a nucleotide sequence shown in SEQ ID NO: 1, or a nucleotide sequence that differs from the nucleotide sequence shown in SEQ ID NO: 1 by deletion, substitution, insertion and/or addition of one to several nucleotides, and a nucleotide sequence shown in SEQ ID NO: 2, or a nucleotide sequence that differs from the nucleotide sequence shown in SEQ ID NO: 2 by deletion, substitution, insertion and/or addition of one to several nucleotides. As another example, the stem part of the shRNA comprises a nucleotide sequence shown in SEQ ID NO: 3, or a nucleotide sequence that differs from the nucleotide sequence shown in SEQ ID NO: 3 by deletion, substitution, insertion and/or addition of one to several nucleotides, and a nucleotide sequence shown in SEQ ID NO: 4, or a nucleotide sequence that differs from the nucleotide sequence shown in SEQ ID NO: 4 by deletion, substitution, insertion and/or addition of one to several nucleotides.

The "antisense RNA" is a single-stranded RNA comprising a sequence complementary to the mRNA sequence of a target gene, and forms a double strand with the mRNA in a sequence-dependent manner. The antisense RNA may be, for example, about 10 to 40 bases in length, preferably 13 to 35 bases in length, more preferably 15 to 25 bases in length. The antisense RNA may partially comprise a DNA sequence. When the RNA molecule contained in the pharmaceutical composition of the present disclosure is an antisense RNA, the antisense RNA preferably comprises a nucleotide sequence complementary to the mRNA sequence of a USAG-1 gene or a partial sequence thereof. As an example, the antisense RNA comprises a nucleotide sequence shown in SEQ ID NO: 2, or a nucleotide sequence that differs from the nucleotide sequence shown in SEQ ID NO: 2 by deletion, substitution, insertion and/or addition of one to several nucleotides. As another example, the antisense RNA comprises a nucleotide sequence shown in SEQ ID NO: 4, or a nucleotide sequence that differs from the nucleotide sequence shown in SEQ ID NO: 4 by deletion, substitution, insertion and/or addition of one to several nucleotides.

The "miRNA" is a single-stranded non-coding RNA, and mainly binds to the 3' untranslated region of a mRNA. The miRNA may be, for example, about 15 to 40 bases in length, preferably 18 to 35 bases in length, and more preferably 20 to 25 bases in length. When the RNA molecule contained in the pharmaceutical composition of the present disclosure is a miRNA, the miRNA preferably comprises a nucleotide sequence complementary to the 3' untranslated region of the mRNA sequence of a USAG-1 gene. In cells, the miRNA is transcribed from a DNA as a pri-miRNA of a hairpin structure comprising a miRNA sequence (guide strand) and a complementary sequence thereof (passenger strand). A part of the pri-miRNA is then cleaved by an enzyme to be released as a pre-miRNA out of nuclei. Then, the pre-miRNA is further cleaved to generate a double-stranded RNA consisting of the guide strand and the passenger strand. Finally, the passenger strand is removed, and the guide strand functions as the miRNA. Therefore, in the pharmaceutical composition of the present disclosure, the miRNA may be present in the form of a hairpin structure such as pri-miRNA or pre-miRNA, or a double-stranded RNA.

The "ribozyme" is a single-stranded RNA having catalytic activity. There are ribozymes having various activities. In the present disclosure, ribozymes capable of recognizing and cleaving a specific nucleotide sequence of a mRNA are preferably used. Hammerhead ribozymes, hairpin ribozymes and the like are known. Based on known techniques in the art, a ribozyme that recognizes and cleaves a desired sequence can be designed and produced. When the RNA molecule contained in the pharmaceutical composition of the present disclosure is a ribozyme, the ribozyme preferably comprises a nucleotide sequence complementary to a part of the mRNA sequence of a USAG-1 gene.

As used herein, the terms "same" and "complementary" mean not only being completely the same as and complementary to a target mRNA sequence, but also being substantially the same as and complementary to the target mRNA sequence as long as a desired action is provided. Thus, when the terms "same" and "complementary" are used herein, the same and complementary nucleotide sequences may comprise deletion, substitution, insertion and/or addition of one to several nucleotides as long as a desired action is provided, as compared to nucleotide sequences that are completely the same as and complementary to the target mRNA sequence.

As used herein, the term "several" refers to about 2 to 6, for example 2 to 5, preferably 2 to 4, for example 3.

The "deletion, substitution, insertion and/or addition" of one to several nucleotides may be deletion, substitution, insertion and/or addition of continuous nucleotides or individually scattered nucleotides. When three or more nucleotides are deleted, substituted, inserted and/or added, a part of the nucleotides may be continuous and the remaining nucleotides may be scattered individually. As used herein, the phrase "a nucleotide sequence that differs by deletion, substitution, insertion and/or addition of one to several nucleotides" means a nucleotide sequence that differs from the target sequence by deletion, substitution, insertion and/or addition of one to several nucleotides as long as a desired action is provided.

The RNA molecule contained in the pharmaceutical composition of the present disclosure may be the RNA molecule itself or a nucleic acid molecule that produces the RNA molecule. Examples of the nucleic acid molecule that produces the RNA molecule include a precursor RNA molecule that produces the desired RNA molecule by processing (for example, splicing, editing, etc.) or the like. Examples of the precursor RNA molecule include, but not limited to, a shRNA which is a precursor of a siRNA, and a pri-miRNA, a pre-miRNA and a double-stranded miRNA which are precursors of a miRNA. Further examples of the nucleic acid molecule that produces the RNA molecule include a DNA and an RNA which are inserted into recombinant vectors, wherein the desired RNA molecule is expressed from a transcription unit containing the DNA or RNA inserted into the vector after administration of the pharmaceutical composition of the present disclosure. The vector may be a vector that provides transient expression of the RNA molecule, or a vector that provides stable expression of the RNA molecule. Examples of the vector include a DNA vector and an RNA vector, and examples thereof include a plasmid vector, and a viral vector such as an adenovirus vector, an adeno-associated virus vector, and a retrovirus vector. In the case of using a DNA vector, a DNA molecule having a nucleotide sequence corresponding to the RNA molecule is inserted into the vector. In the case of using an RNA vector, for example, a DNA construct is generated by reverse transcription from the RNA vector after administration, the DNA construct is incorporated into cell chromosomes, and the RNA molecule is transcribed from the incorporated DNA construct. Vectors for expressing RNA molecules have been well studied in the art, and a person skilled in the art can appropriately construct the vectors using known techniques and/or commercially available kits.

The pharmaceutically acceptable carrier contained in the pharmaceutical composition of the present disclosure may be any carrier used for preparations to be topically administered. Preferably, a carrier suitable for delivery of a nucleic acid molecule is used. Preferred examples of the pharmaceutically acceptable carrier contained in the pharmaceutical composition of the present disclosure include, but not limited to, gelatin, gelatin hydrogel, polylactic acid, a poly(lactic acid-co-glycolic acid) copolymer (PLGA), an in situ gelling system comprising polymethacrylic acid (PMA) and polyethylene glycol (PEG) and hydroxypropylmethylcellulose, a poly(lactic acid)-poly(ethylene glycol) copolymer (PLA-PEG), poly(2-aminoethyl propylene phosphate), poly(α-(4-aminobutyl)-L-glycolic acid) (PAGA), a poloxamer, an ethylene-co-vinyl acetate copolymer (EVAc), a silk elastin-like polymer (SELP), collagen, denatured collagen-PLGA, atelocollagen, a liposome, a nanoparticle comprising cationic dextrin, a nanoparticle comprising PEG, a nanoparticle comprising transferrin, and a nanoparticle comprising adamantan. Examples of the liposome include a cationic liposome, an anionic liposome, a PEG-modified liposome, and a neutral liposome. Various liposomes that were developed for delivery of nucleic acid molecules can be used.

Further preferred examples of the pharmaceutically acceptable carrier include cationized gelatin and cationized gelatin hydrogel. The cationized gelatin can be prepared, for example, by introducing an amino group into gelatin. The cationized gelatin may be prepared, for example, by chemically introducing ethylenediamine into the carboxyl group of gelatin. Hydrogel can be prepared by crosslinking gelatin, and the cationized gelatin hydrogel can be prepared by crosslinking the cationized gelatin. The gelatin or the cationized gelatin can be chemically crosslinked, for example, by using glutaraldehyde or the like. The cationized gelatin may also form microspheres. When the cationized gelatin microspheres are used, the RNA molecule may be impregnated and fixed in the microspheres.

In the pharmaceutical composition of the present disclosure, the RNA molecule is supported on the pharmaceutically acceptable carrier. A method for supporting the RNA molecule is not particularly limited. For example, the RNA molecule may be encapsulated in or impregnated in the carrier, or the RNA molecule may form a conjugate or a complex with the carrier. Since the RNA molecule is supported on the carrier in the pharmaceutical composition of the present disclosure, the RNA molecule is stabilized, and maintained in the body of a subject without being decomposed even after administration to the subject. In addition, since the RNA molecule is supported on the carrier, the RNA molecule can be sustainedly released at an administration site.

The pharmaceutical composition of the present disclosure may further contain additives such as pharmaceutically acceptable stabilizers and excipients, if necessary.

The pharmaceutical composition of the present disclosure may be in any form suitable for local administration, and examples thereof include an injection and the like. A person skilled in the art can appropriately select the carrier and the additive based on the administration form and the like of the pharmaceutical composition, and formulate them by a conventional method.

The pharmaceutical composition of the present disclosure is locally administered to, for example, a tooth missing site or a tooth formation site, or to, for example, a mandible. The pharmaceutical composition of the present disclosure is preferably administered within a maxilla or mandible. BMP and WNT signaling which USAG-1 is involved in plays an important role in life activity. Since the pharmaceutical composition of the present disclosure inhibits the USAG-1 production itself, there is a concern that systemic administration of the pharmaceutical composition may cause side effects on sites other than a tooth missing site. Thus, local administration to a tooth missing site or tooth formation site permits the pharmaceutical composition of the present disclosure to provide tooth regeneration without serious side effects.

An administration amount of the pharmaceutical composition of the present disclosure is not particularly limited. The pharmaceutical composition of the present disclosure may be administered in an amount sufficient to inhibit the expression of a USAG-1 gene, and such an amount can be appropriately determined by a person skilled in the art.

A subject to which the pharmaceutical composition of the present disclosure is administered includes a mammal, for example, a human, a dog, a cat, a horse, a rat, a ferrule, a porcine, a monkey, or the like, and is preferably a human.

As used herein, the "tooth regeneration therapy" includes treatment of congenital and acquired tooth loss, for example, treatment of congenital and acquired tooth agenesis. The "tooth regeneration" includes, for example, regeneration of a missing tooth (recovery of a missing tooth) and formation of a new tooth such as the third dentition. The congenital tooth agenesis that can be treated with the pharmaceutical composition of the present disclosure is not particularly limited, and may be congenital tooth agenesis due to any causative gene. Examples of congenital tooth agenesis that can be treated with the pharmaceutical composition of the present disclosure include, but not limited to, congenital tooth agenesis whose causative gene is RUNX2, MSX1, EDA, WNT10A, PAX9, or AXIN2, preferably congenital tooth agenesis whose causative gene is RUNX2.

A further aspect of the present invention provides a method for tooth regeneration, the method comprising locally administering an RNA molecule that targets an USAG-1 gene and inhibits the expression of USAG-1 together with a pharmaceutically acceptable carrier to a subject in need thereof. The pharmaceutical composition of the present disclosure can be used as the RNA molecule and the carrier. The subject in need thereof is a subject losing a tooth, and examples of the subject include the above-mentioned mammals. The amount to be administered and the tooth regeneration therapy are as described for the pharmaceutical composition of the present disclosure.

### Examples

Hereinafter, the present invention will be explained in more detail with reference to Examples which the present invention is not limited to.

### Example 1: USAG-1 gene knockdown by mouse siRNA

### (1) USAG-1 gene knockdown using siRNA targeting USAG-1 gene

Two types of mouse USAG-1-targeting stealth siRNAs (#304, #903) were constructed (manufactured by Thermo Fisher Scientific, Waltham, Massachusetts, USA). The sense and antisense strand sequences of stealth siRNA #304 and #903 are shown in Table 1.

**[Table 1]**

| Table 1. Mouse USAG-1 knockdown Stealth siRNA | | | |
|---|---|---|---|
| | Sense strand | Antisense strand | NCBI accession No. |
| #304 | UCAGUAGCACUGGACUGGAU CGAAA (SEQ ID NO: 5) | UUUCGAUCCAGUCCAGUGC UACUGA (SEQ ID NO: 6) | NM_025312 |
| #903 | CAAGUGUCUCAAGAUGUAAU GAGUA (SEQ ID NO: 7) | UACUCAUUACAUCUUGAGA CACUUG (SEQ ID NO: 8) | NM_025312 |

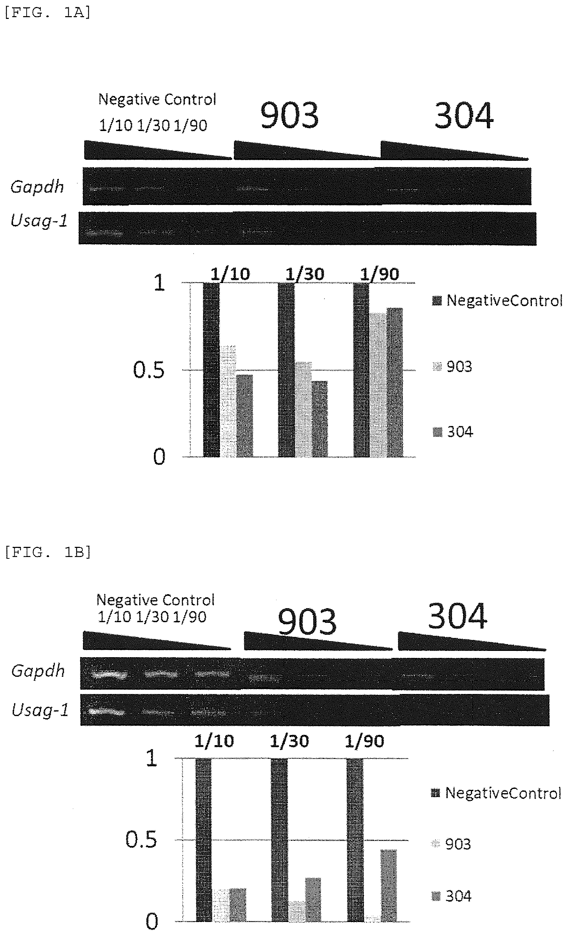

In mouse enamel epithelial cells (mHAT9d cells) in which mouse USAG-1 expression was found, efficiency of USAG-1 gene knockdown was determined by semi-quantitative (sq) PCR using stealth siRNAs #304 and #903. Specifically, a mRNA was extracted from the mHAT9d cells that were cultured in a medium containing each siRNA (negative control, #304, #903) by a conventional method, and then, a single-stranded cDNA was synthesized from the mRNA using reverse transcriptase by a conventional method. Serial dilutions (1/10, 1/30 and 1/90) of the single-stranded cDNA were prepared. Using each dilution, PCR amplification for Gapdh and Usag-1 gene regions was performed to determine the knockdown effect on the mouse USAG-1 gene. Results are shown in FIG. 1A. As a negation control, Stealth RNAi TM siRNA Negative Control Med GC Dup (manufactured by ThermoFisher Scientific, Waltham, Massachusetts, USA) was used.

In a mandibular organ culture of an embryonic day 10 (E10) wild-type mouse, USAG-1 gene knockdown effect was determined by sqPCR using stealth siRNAs #304 and #903. Specifically, a mRNA was extracted from the mHAT9d cells that were cultured in a medium containing each siRNA (negative control, #304, #903) by a conventional method, and then, a single-stranded cDNA was synthesized from the mRNA using reverse transcriptase by a conventional method. Serial dilutions (1/10, 1/30 and 1/90) of the single-stranded cDNA were prepared. Using each dilution, PCR amplification for Gapdh and Usag-1 gene regions was performed to determine the knockdown effect on the mouse USAG-1 gene. Results are shown in FIG. 1B.

As seen from FIG. 1A and FIG. 1B, the USAG-1 gene knockdown effect was shown when either of the two types of siRNA tested was used.

### (2) Effect of knockdown using siRNA on developmental stage of tooth germ and tooth germ number

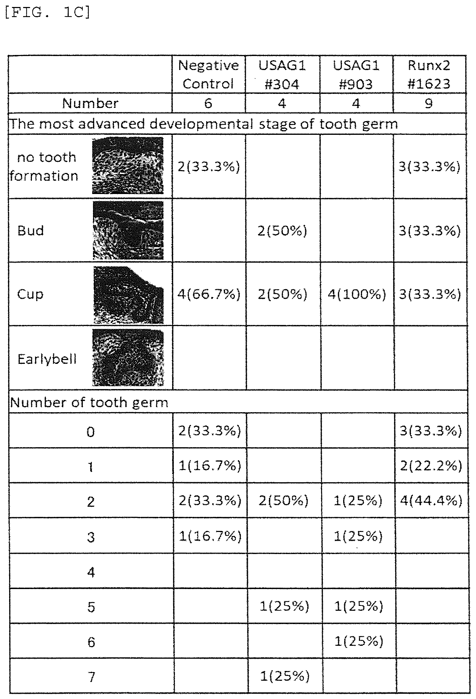

The effects of USAG-1 gene knockdown and Runx2 gene knockdown on the development stage of tooth germs and the number of tooth germs in an E10 wild-type mouse mandibular organ culture were investigated using stealth siRNAs #304 and #903 targeting mouse USAG-1 and a stealth siRNA targeting mouse Runx2 (#1623) (Table 2, Saito K, et al., Sci. Rep., 2018) . Specifically, a mRNA was extracted from the mandibular organ culture that was cultured in a medium containing each siRNA (negative control, #304, #903, #1623) by a conventional method, and then, a single-stranded cDNA was synthesized from the mRNA using reverse transcriptase by a conventional method. Serial dilutions (1/10, 1/30 and 1/90) of the single-stranded cDNA were prepared. Using each dilution, PCR amplification for Gapdh and Usag-1 gene regions was performed to determine the knockdown effect on the mouse USAG-1 gene. Results are shown in FIG. 1C.

**[Table 2]**

| Table 2. Mouse Runx2 knockdown Stealth siRNA | | |
|---|---|---|
| | Sense strand | Antisense strand |
| #1623 | GGCCACUUACCACAGAGCUAUUA AA (SEQ ID NO: 9) | UUUAAUAGCUCUGUGGUAAGUGG CC (SEQ ID NO: 10) |

As seen from FIG. 1C, there was no significant change in the tooth development stage by administration of stealth siRNAs #903 and #304 targeting USAG-1 and stealth siRNA #1623 targeting Runx2 as compared to the negative control. It was found that administration of stealth siRNA #304 targeting USAG-1 tended to increase the number of tooth germs, and administration of stealth siRNA #1623 targeting Runx2 tended to decrease the number of tooth germs. These results show that the knockdown of USAG-1 and Runx2 genes by siRNAs led to promotion of tooth development and induction of tooth loss, respectively, similarly to the knockout effects of these genes that were previously found by the inventors.

### Example 2: Drug delivery of siRNA by carrier

This Example was performed to confirm that a cationized gelatin hydrogel carrier functions as a drug delivery system (DDS) for local administration of USAG-1 siRNA.

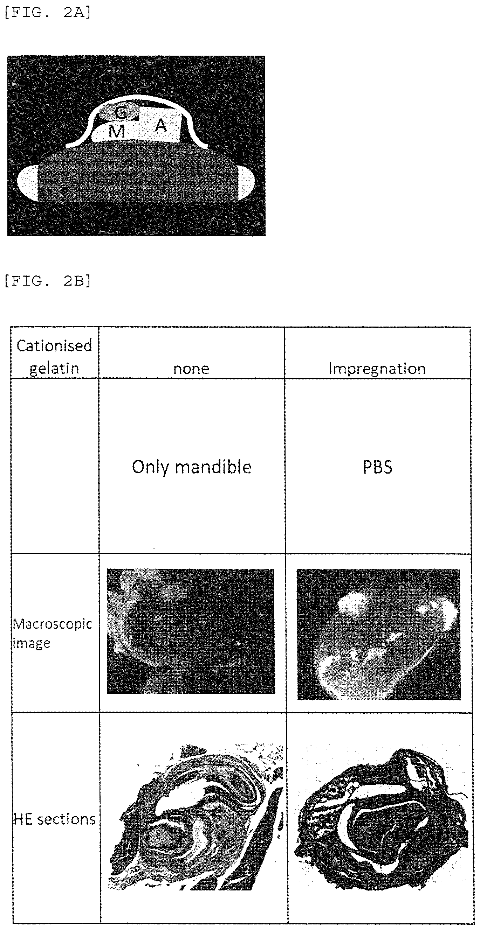

First, a subrenal capsule assay was performed using, as a control, a half of an E10 wild-type mouse mandible together with a cationized gelatin hydrogel sheet impregnated with PBS or without any carrier (i.e. only the mandible was transplanted). The subrenal capsule assay was performed as described later. Nineteen days after transplantation, tissue attached to the mouse kidney was collected. The tissue section histologically contained a tooth structure (FIG. 2B). No difference in growth was detected between the presence and absence of the cationized gelatin hydrogel (FIG. 2B). These results show that the cationized gelatin has no apparent toxicity to tooth development.

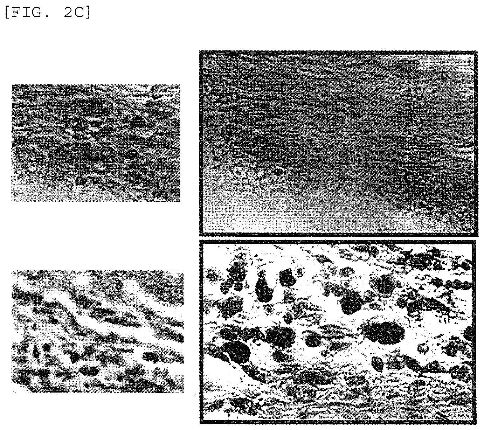

Next, a subrenal capsule assay was performed using an E10 wild-type mouse mandible together with a cationized gelatin hydrogel sheet impregnated with siRNA labeled with Alexa Fluor488. The subrenal capsule assay was performed as described later. Ten days after the subrenal capsule assay, tissue sections were collected and then evaluated by HE staining and immunostaining with an anti-Alexa Fluor488 antibody (FIG. 2C). As a result, a large number of positive cells were observed in the immunostained sections, and thus the siRNA labeled with Alexa Fluor488 was found to be contained in the sections. These results show that the cationized gelatin hydrogel can control the release of siRNA into tooth germs (can lead to the sustained release of siRNA into tooth germs) and is effective as a DDS for topical administration of siRNA in the subrenal capsule assay of the E10 mouse mandible.

### Protocol for subrenal capsule assay

A lateral mandible of an E10 wild-type mouse was incised under a stereomicroscope. The explant was transplanted to the subcapsular space of the kidney of a nude mouse (KSN/Slc) together with a cationized gelatin hydrogel sheet (FIG. 2A).

The cationized gelatin was prepared as previously described (for example, J Control Release. 2005 Jul 20; 105(3): 318-31). A solution of the cationized gelatin was lyophilized to prepare a cationized gelatin sheet. The cationized gelatin sheet was crosslinked at 160°C for 24 hours. The cationized gelatin hydrogel sheet thus obtained was weighed by an electronic scale to be divided into 1 mg. To impregnate the cationized gelatin hydrogel sheet with PBS or stealth siRNA, an aqueous solution containing PBS or 10 µL of the stealth siRNA was added dropwise onto 1 mg of the crosslinked cationized gelatin hydrogel sheet and then incubated at 37°C for 1 hour. The concentration of the stealth siRNA was adjusted to 322 µg/mL using PBS.

The subrenal capsule transplantation was performed under a stereomicroscope using fine tweezers and a scalpel for microsurgery. Ten days, 15 days, and 19 days after the transplantation, mice were euthanized and kidneys were removed. Tissue attached to the kidney was collected, fixed in 4% paraformaldehyde, embedded in paraffin, continuously sectioned (each 7 µm), and then subjected to hematoxylin and eosin staining (HE staining).

### Example 3: Recovery from knockdown

A mandibular explant from an E10 wild-type mouse was transplanted together with a cationized gelatin hydrogel impregnated with stealth siRNA #304 targeting USAG-1 and/or stealth siRNA #1623 targeting Runx2 to the subcapsular space of the kidney of a nude mouse (KSN/Slc), according to the above-described protocol for subrenal capsule assay. Nineteen days after transplantation, sections of tissue attached to the kidney were obtained. The number of tooth germs formed in each explant sample was counted. HE staining, µCT imaging and 3D reconstruction of the HE sections were performed.

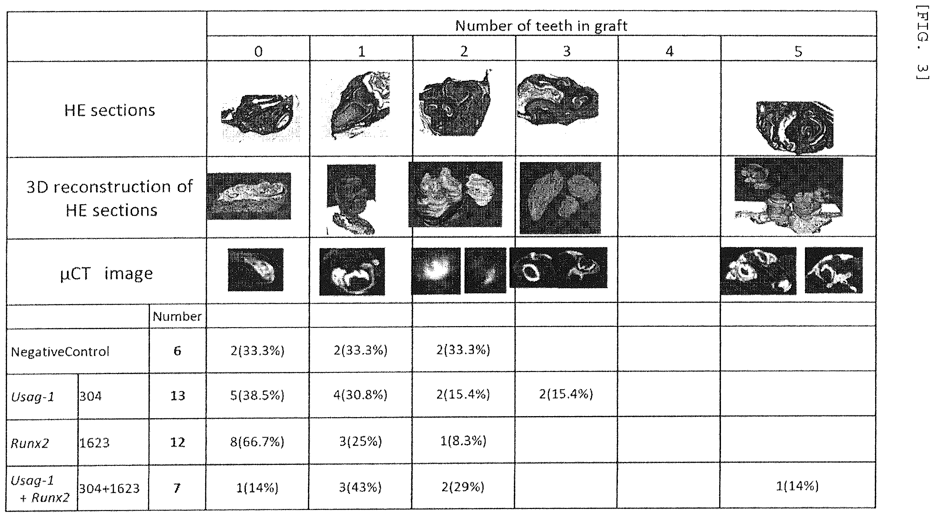

Results are shown in FIG. 3. In FIG. 3, "Number" indicates the number of mandibles transplanted together with each siRNA to the subcapsular space of the kidney. "Number of teeth in graft" indicates the number of samples having each number of tooth germs. As a result, a decreased number of teeth caused by administration of stealth siRNA targeting Runx2 was recovered by simultaneous administration of stealth siRNA #304 targeting USAG-1. Thus, it was found that the double knockdown of USAG-1 and Runx2 genes by siRNAs led to recovery of tooth formation, similarly to the double knockout of these genes previously found by the inventors.

### Example 4: Recovery from knockout

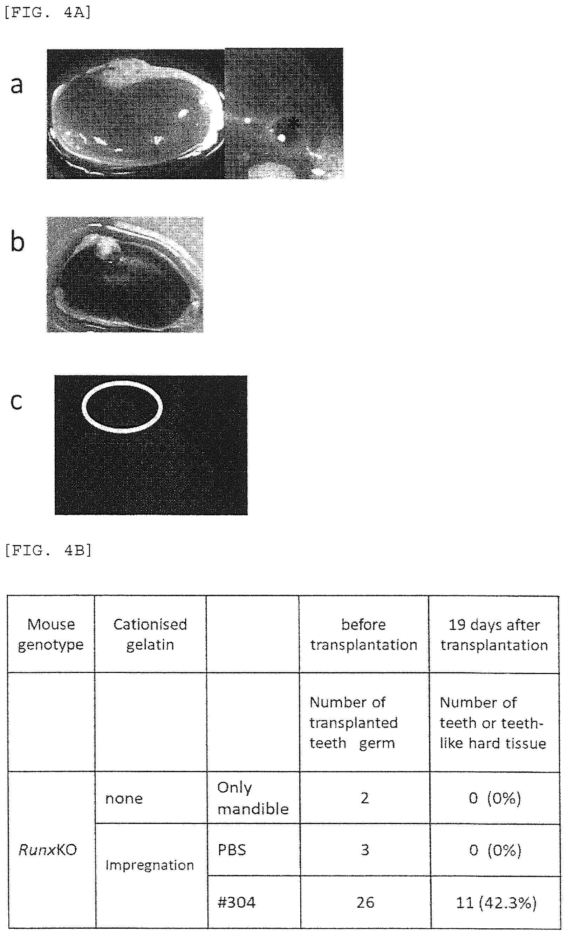

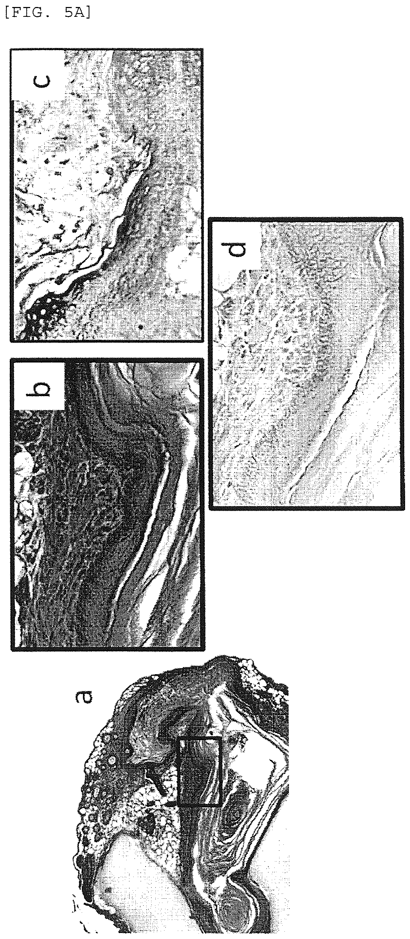

A mandibular explant from an E10 Runx2-knockout (KO) mouse was transplanted together with a cationized gelatin hydrogel impregnated with stealth siRNA #304 targeting USAG-1 to the subcapsular space of the kidney of a nude mouse (KSN/Slc), according to the above-described protocol for subrenal capsule assay. As a control, a cationized gelatin hydrogel impregnated with PBS or the siRNA without any carrier was used. Nineteen days after transplantation, sections of tissue attached to the kidney were obtained. The number of tooth germs or tooth-like tissue formed in each explant sample was counted. HE staining and µCT imaging were performed. Results are shown in FIG. 4A, FIG. 4B, and "a" and "b" of FIG. 5A ("b" is a magnified view of a portion surrounded by a square in "a").

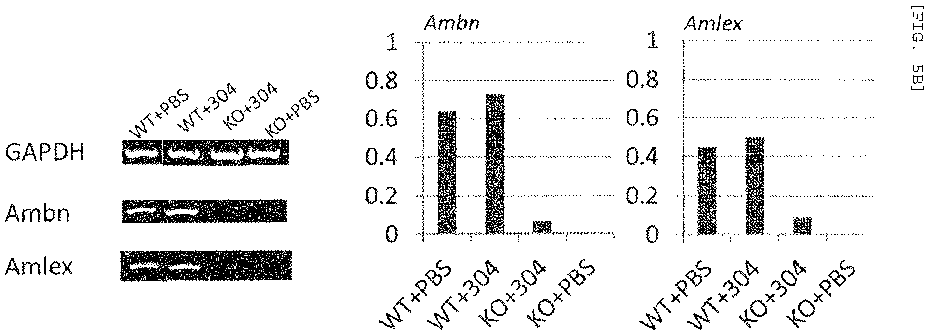

Furthermore, immunostaining of amelogenin, which is a protein specific for tooth enamel, was performed. For the immunostaining, a rabbit anti-amelogenin polyclonal antibody (manufactured by HOKUDO) was used as a primary antibody, and an anti-rabbit antibody was used as a secondary antibody. For a negative control, staining was performed without the primary antibody. Furthermore, the obtained tissue sample was homogenized using a TRIZOL (registered trademark) reagent (manufactured by Thermo Fisher Scientific). For determining the expression of mRNAs of amelogenin and ameloblastin using complementary DNAs synthesized from the sample, the gene expression levels were measured by RT-PCR. Similarly, the expression levels of mRNAs of amelogenin and ameloblastin were determined using the wild-type mouse mandibular explant sample transplanted together with stealth siRNA #304 or a cationized gelatin hydrogel impregnated with PBS as obtained by Example 3. Results are shown in "c" and "d" of FIG. 5A (c: immunostaining of amelogenin, d: negative control; "c" and "d" are magnified views of a portion surrounded by a square in FIG. 5A-a) and FIG. 5B.

When the UASG-1 gene was knockdown by siRNA, tooth-like structures were macroscopically observed in about half of the transplanted tooth germs ("a" and "b" of FIG. 4A, and FIG. 4B). Although no apparent calcification was observed by µCT ("c" of FIG. 4A), the possibility of differentiation of ameloblasts which is indicative of differentiation of tooth germs was histologically found ("a" and "b" of FIG. 5A). As a result of immunostaining of amelogenin which is a protein specific for enamel, a positive image was observed ("c" of FIG. 5A). Furthermore, the mRNA expression levels of amelogenin (Ambn) and ameloblastin (Amlex) which is an early protein specific for enamel were determined by RT-PCR (FIG. 5B). As a result, the mRNA expression of amelogenin and ameloblastin was found.

### Example 5: USAG-1 gene knockdown by human siRNA

Two types of stealth siRNAs (#1706, #1347) targeting human USAG-1 were constructed (manufactured by Thermo Fisher Scientific). The sense and antisense strand sequences of stealth siRNA #1706 and #1347 are shown in Table 3.

**[Table 3]**

| Table 3. Human USAG-1 knockdown Stealth siRNA | | |
|---|---|---|
| | Sense strand | Antisense strand |
| #1706 | CCUCUAACAUGUAUAAAGGGUGC AA (SEQ ID NO: 1) | UUGCACCCUUUAUACAUGUUAGA GG (SEQ ID NO: 2) |
| #1347 | AUUUGUCAAUGUUAUUAUUUAAU GA (SEQ ID NO: 3) | UCAUUAAAUAAUAACAUUGACAA AU (SEQ ID NO: 4) |

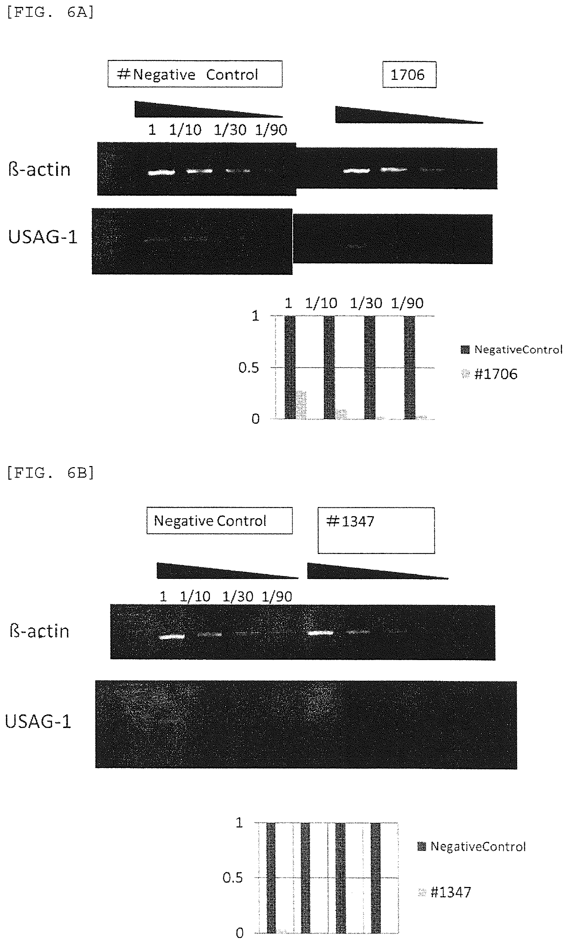

In human embryonic kidney cells (HEK293 cells) in which human USAG-1 expression was found, efficiency of USAG-1 gene knockdown was determined by sqPCR using stealth siRNAs #1706 and #1347 in the same manner as in Example 1-(1). Results are shown in FIG. 6A and FIG. 6B. As seen from FIG. 6A and FIG. 6B, the USAG-1 gene knockdown effect was shown when either of the two types of siRNA tested was used.

### Industrial Applicability

The RNA molecule targeting USAG-1 can be used for the treatment of congenital tooth agenesis and acquired tooth loss. In addition, the RNA molecule targeting USAG-1 is effective for the formation of the third dentition. Thus, the RNA molecule targeting USAG-1 leads to development of a molecular-targeted drug for tooth regeneration in the pharmaceutical field and establishment of a dental regenerative therapy based on formation of the third dentition.

## Claims

1. A pharmaceutical composition for topical administration for tooth regeneration therapy, comprising an RNA molecule targeting USAG-1 or a nucleic acid molecule that produces the RNA molecule, and a pharmaceutically acceptable carrier.

2. The pharmaceutical composition according to claim 1, wherein the RNA molecule is selected from the group consisting of a siRNA, a shRNA, an antisense RNA, a miRNA, and a ribozyme.

3. The pharmaceutical composition according to claim 2, wherein the RNA molecule is a siRNA or a shRNA.

4. The pharmaceutical composition according to claim 3, wherein the siRNA or shRNA comprises:
(1) a sense strand comprising a nucleotide sequence shown in SEQ ID NO: 1, or a nucleotide sequence that differs from the nucleotide sequence shown in SEQ ID NO: 1 by deletion, substitution, insertion and/or addition of one to several nucleotides, and an antisense strand comprising a nucleotide sequence shown in SEQ ID NO: 2, or a nucleotide sequence that differs from the nucleotide sequence shown in SEQ ID NO: 2 by deletion, substitution, insertion and/or addition of one to several nucleotides, or
(2) a sense strand comprising a nucleotide sequence shown in SEQ ID NO: 3, or a nucleotide sequence that differs from the nucleotide sequence shown in SEQ ID NO: 3 by deletion, substitution, insertion and/or addition of one to several nucleotides, and an antisense strand comprising a nucleotide sequence shown in SEQ ID NO: 4, or a nucleotide sequence that differs from the nucleotide sequence shown in SEQ ID NO: 4 by deletion, substitution, insertion and/or addition of one to several nucleotides.

5. The pharmaceutical composition according to claim 2, wherein the RNA molecule is an antisense RNA.

6. The pharmaceutical composition according to claim 5, wherein the antisense RNA comprises:
(1) a nucleotide sequence shown in SEQ ID NO: 2, or a nucleotide sequence that differs from the nucleotide sequence shown in SEQ ID NO: 2 by deletion, substitution, insertion and/or addition of one to several nucleotides, or
(2) a nucleotide sequence shown in SEQ ID NO: 4, or a nucleotide sequence that differs from the nucleotide sequence shown in SEQ ID NO: 4 by deletion, substitution, insertion and/or addition of one to several nucleotides.

7. The pharmaceutical composition according to any one of claims 1 to 6, wherein the carrier is selected from the group consisting of collagen, gelatin, gelatin hydrogel, polylactic acid, a poly(lactic acid-co-glycolic acid) copolymer (PLGA), an *in situ* gelling system comprising polymethacrylic acid (PMA) and polyethylene glycol (PEG) and hydroxypropylmethylcellulose, a poly(lactic acid)-poly(ethylene glycol) copolymer (PLA-PEG), poly(2-aminoethyl propylene phosphate), poly(α-(4-aminobutyl)-L-glycolic acid) (PAGA), a poloxamer, an ethylene-co-vinyl acetate copolymer (EVAc), a silk elastin-like polymer (SELP), denatured collagen-PLGA, atelocollagen, a liposome, a nanoparticle comprising cationic dextrin, a nanoparticle comprising PEG, a nanoparticle comprising transferrin, and a nanoparticle comprising adamantan.

8. The pharmaceutical composition according to claim 7, wherein the carrier is cationized gelatin, cationized gelatin hydrogel, or a cationized gelatin microsphere.

9. The pharmaceutical composition according to any one of claims 1 to 8, wherein the tooth regeneration therapy is a treatment of congenital or acquired tooth agenesis, or regeneration of a missing tooth.
